Europäisches Patentamt

European Patent Office

Office européen des brevets

(19)

(11) Publication number: 0 401 006 B1

# EUROPEAN PATENT SPECIFICATION

(45) Date of publication of patent specification: 06.12.95    (51) Int. Cl.⁶: **C12P 21/08**, G01N 33/74

(21) Application number: 90305906.1

(22) Date of filing: 31.05.90

The file contains technical information submitted after the application was filed and not included in this specification

(54) hANP antibody and method for immunological determination of hANP.

(30) Priority: 31.05.89 JP 139474/89

(43) Date of publication of application:
05.12.90 Bulletin 90/49

(45) Publication of the grant of the patent:
06.12.95 Bulletin 95/49

(84) Designated Contracting States:
**GB**

(56) References cited:

**FEBS LETTERS, vol. 218, no. 1, June 1987, Elsevier Science Publishers B.V., Amsterdam (NL); G. THEISS et al., pp. 159-162**

**LIFE SCIENCES, vol. 43, no. 9, September 1988, Pergamon Press Plc, Elmsford, NY (US); S. NAOMI et al., pp. 761-768**

**KLINISCHE WOCHENSCHRIFT, vol. 65, 1987, Springer-Verlag (DE); J.-P. STASCH et al., pp. 533-537**

(73) Proprietor: **SUNTORY LIMITED**
**1-40, Dojimahama 2-chome**
**Kita-ku,**
**Osaka-shi,**
**Osaka 530 (JP)**

(72) Inventor: **Miura, Hiroshi, c/o Sakura Fact.**
**Green Cross Corporation,**
**8-5, Osaku-1-chome**
**Sakura-shi (JP)**
Inventor: **Nogami, Toshihiko c/o Sakura Fact.**
**Green Cross Corporation,**
**8-5, Osaku-1-chome**
**Sakura-shi (JP)**
Inventor: **Cho, Sontesu, c/o Sakura Fact.**
**Green Cross Corporation,**
**8-5, Osaku-1-chome**
**Sakura-shi (JP)**
Inventor: **Ohmi,Shoichi, c/o Sakura Fact.**
**Green Cross Corporation,**
**8-5, Osaku-1-chome**
**Sakura-shi (JP)**
Inventor: **Kazahaya, Yasuhiro, c/o Sakura**
**Fact.**
**Green Cross Corporation,**
**8-5, Osaku-1-chome**
**Sakura-shi (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Inventor: **Hayashi, Yujiro, c/o Iyaku Senta of Suntory Ltd.**
**2716-1, Kurakake,**
**Akaiwa**
**Chiyodamachi,**
**Ora-gun,**
**Gunma-ken (JP)**
Inventor: **Terano, Yoshitake, c/o Seibutsu Igaku Kenkyusho**
**of Suntory Limited,**
**1-1 Wakayamadai-1-chome**
**Shimamotocho,**
**Mishima-gun,**
**Osaka (JP)**

(74) Representative: **Coleiro, Raymond et al**
**MEWBURN ELLIS**
**York House**
**23 Kingsway**
**London WC2B 6HP (GB)**

EP 0 401 006 B1

**Description**

BACKGROUND OF THE INVENTION

Field of the Invention

The present invention relates to a human atrial natriuretic polypeptide (hereafter abbreviated as hANP) antibody and a method for immunological determination of hANP using the hANP antibody.

Related Art Statement

An atrial natriuretic polypeptide (hereafter abbreviated as ANP) is a hormone which is secreted from atrium and circulates in blood. It has been found that ANP plays an important role in regulating blood pressure and controlling the volume of body fluid in a mammalian body. In addition to that ANP acts on the renal circulatory systems, there have been findings suggesting the possibility that ANP would be present also in the brain and would function as a neurotrophic peptide.

According to isolation and purification of ANP from autopsy tissue of human atrium, it has been revealed that 3 kinds of hANP, i.e., $\alpha$ type having a molecular weight of about 3 KDa and $\beta$ type having a molecular weight of about 6 KDa and $\gamma$ type having a molecular weight of 13 KDa, are present in human atrium. The chemical structures of these ANPs have also been determined.

$\alpha$ Type hANP (hereafter abbreviated as $\alpha$-hANP) is a single-chain peptide composed of 28 amino acids and contains an S-S bond in its molecule. $\alpha$-hANP is the main component of ANP in blood.

$\beta$ Type hANP (hereafter abbreviated as $\beta$-hANP) has a specific structure that is an antiparallel dimer of $\alpha$-hANP and possesses about one-fifth of the diuretic/hypotensive and smooth muscle relaxing activity of $\alpha$-hANP.

$\gamma$ Type hANP (hereafter abbreviated as $\gamma$-hANP) is a high molecular protein composed of 126 amino acids and contains an $\alpha$-hANP structure at the C-terminus (amino acids 99-126).

On the other hand, physiologically active substances have been generally studied for their kinetics on the basis of their activity as an index. This applies not only to ANP but also to all other substances which have been investigated and determined in terms of physiological activity as an index. As such a substance, cytokines may be exemplified. In the case of hANP, the isolation and purification have been performed using its smooth muscle relaxing activity, etc. as an index. However, this method is complicated and requires technical skill. Therefore, it is necessary to develop a method for immunological determination, as an improved method solving such a defect. For such a method, hANP antibody is required and a variety of hANP antibodies are known so far. For example, there may be mentioned CR-3 antibody disclosed in Japanese Patent KOKAI (Laid-Open) No. 61-53299, 23M-D9 antibody disclosed in Japanese Patent KOKAI (Laid-Open) No. 61-227597 and KY-ANP-1 antibody disclosed in Japanese Patent KOKAI (Laid-Open) No. 62-211555, WO 86-6742 and Japanese Patent KOKAI (Laid-Open) No. 64-61500.

However, the known hANP antibodies involve the following problems:

(1) The recognition sites have not been investigated in detail; and

(2) Any of the known hANP antibodies could not simultaneously recognize all of the C-terminus, ring structure and N-terminus in the hANP molecule, so that different measurement data as to hANP properties were obtained depending on difference in their specificities.

Therefore, it is desired to develop an antibody capable of simultaneously recognizing a plurality of physiologically active sites of hANP, namely, at least the disulfide bond and the C-terminal Tyr at 28-position in the hANP molecule.

SUMMARY OF THE INVENTION

It is an object of the present invention to provide a hANP antibody capable of specifically reacting with a specific amino acid sequence and chemical structure in the hANP molecule.

It is another object of the present invention to provide a method for the immunological determination of hANP using this hANP antibody, by which hANP can be detected with high sensitivity.

It is still another object of the present invention to provide a kit for the immunological determination of hANP.

According to one aspect of the present invention, there is provided a hANP antibody, which is capable of recognizing at least Tyr at 28-position, the disulfide bond between Cys at 7-position and Cys at 23-position and the fragment from Cys at 7-position to Met at 12-position in the hANP molecule.

3

According to another aspect of the present invention, there is provided a method for the immunological determination, which uses the hANP antibody.

According to still another aspect of the present invention, there is provided a kit for the immunological determination of hANP, which comprises the hANP antibody.

DETAILED DESCRIPTION OF THE PREFERRED EMBODIMENTS

In the present invention, the hANP antibody can be produced according to a conventional method for producing an antibody. As an immunogen for producing the hANP antibody, antigen per se or hapten antigen is used.

As the antigen, there may be preferably exemplified hANP or its fragments. A preferred example of hANP is α-hANP. α-hANP can be prepared according to a conventional peptide synthesis method in the liquid phase or according to a method as disclosed in Peptide Chemistry, 241 (1984). Commercially available hANP may also be employed. As the hANP fragment, there may be preferably used fragments of amino acids 4-28, 5-28 and 7-28 in an amino acid sequence of α-hANP, which fragments are abbreviated as α-hANP (4-28), α-hANP (5-28), α-hANP (7-28), respectively. The amino acid sequence of α-hANP has been reported by Kangawa et al., Biochem. Biophys. Res. Commun., 118, 131-139 (1984). This literature is referred to herein.

In the case of hapten antigen, antigen bound to a carrier may be used as the immunogen. Known carriers are used and such examples include serum albumins such as bovine serum albumin, horse serum albumin, rabbit serum albumin, sheep serum albumin and human serum albumin; globulins such as bovine serum globulin, horse serum globulin, rabbit serum globulin and human serum globulin; thyroglobulins such as bovine thyroglobulin, horse thyroglobulin, rabbit thyroglobulin and human thyroglobulin; hemoglobins such as bovine hemoglobin, horse hemoglobin and human hemoglobin; and hemocyanins such as Keyhole limpet hemocyanin.

For preparation of hapten antigen, conventional methods may be used and such examples include a method for binding antigen to a carrier using a binder; and the like. As the binder used herein, there may be known dialdehydes such as glutaraldehyde, malonyldialdehyde and succinylaldehyde; dimaleimides such as N,N'-o-phenylene dimaleimide and N,N'-m-phenylenedimaleimide; carbodiimides such as dicyclohexylcarbodiimide and N-ethyl-N'-(3-dimethylaminopropyl)carbodiimide [cf., for example, Biohem. Biophys. Res. Commun., 129, 248-255 (1984)].

The hANP antibody of the present invention may be either polyclonal antibody or monoclonal antibody.

The polyclonal hANP antibody can be obtained by immunizing an animal with highly purified hANP or its fragment and recovering and purifying the polyclonal hANP antibody as an antiserum from the serum.

The serum may be prepared by a conventional method. For example, an emulsion mixture of highly purified hANP and Freund's complete adjuvant is prepared and intracutaneously injected to animal 2 or 3 times at intervals. Several days after the final booster, blood is collected and coagulated at room temperature and then allowed to stand at 4°C overnight. Centrifugation at 3,000 rpm for 20 minutes gives antiserum, i.e., a hANP antibody.

As the animal used for the immunization, its species is not particularly limited but rat, mouse, rabbit, goat and horse may be used. After defatting the serum with, e.g., trichlorotrifluoroethane for clarification, the antiserum may be purified according to a method as described in J. Am. Chem. Soc., 62, 3386 (1940) or Fed. Proc., 17, 1161 (1958).

The monoclonal hANP antibody can be obtained according to a conventional cell fusion method. The cell fusion method may be performed in a conventional manner and one example comprises reacting cells having a proliferation property and lymphocytes capable of producing the desired antibody in the presence of polyethylene glycol thereby to produce hybrid cells having both proliferation property and antibody productivity. The antibody produced by the hybrid cells is a monoclonal antibody capable of reacting only with one antigenic determinant. More specifically, mouse myeloma cells as cells having proliferation property may be fused with spleen cells, i.e., B cells, as antibody-producing lymphocytes from mouse immunized with hANP; the hybrid cell capable of producing the desired antibody is cloned by screening followed by cell culture to obtain the monoclonal hANP antibody.

The recovered antibody is purified by a conventional method. Examples of the purification method include precipitation with ammonium sulfate, a treatment with ion exchangers, gel filtration and affinity chromatography using protein A.

The thus obtained antibody of the present invention can recognize at least three sites of Tyr at 28-position [Tyr (28)], the disulfide bond between Cys at 7-position [Cys (7)] and Cys at 23-position [Cys (23)] and fragment from Cys at 7-position to Met at 12-position [Cys (7) - Met (12)] in the hANP molecule,

4

especially in the $\alpha$-hANP molecule.

Therefore, the antibody of the present invention is capable of reacting with hANP having Tyr (28) at the C-terminus and a cyclic structure in the molecule. Specifically, an antibody embodying the invention reacts with $\alpha$-hANP and $\beta$-hANP. Furthermore, the antibody reacts with $\alpha$-hANP, $\alpha$-hANPs (5-28) and (7-28) but does not react with $\alpha$-hANPs (1-10), (13-28), (18-28) and (5-27). Although the antibody reacts with Met [O][12] type $\alpha$-hANP which is a modified $\alpha$-hANP having an oxidized sulfur atom in Met at 12-position of c-hANP and which has no ANP related activities [Biochem. Biophys. Res. Commun., 128, 538 (1985)], this Met [O][12] type $\alpha$-hANP has been believed hardly to be generated in a physiological condition of a living organism.

An antibody embodying the present invention also has properties of: Ig class, IgG; molecular weight, 150,000 to 160,000; isoelectric point, 5.8 - 7.3; etc.

The antibody of the present invention may be used as a reagent for clinical inspections such as diagnosis of heart diseases or renal disorders, judgment of therapeutic effects, monitoring of electrolytes (especially sodium ions, etc.) and the like.

The antibody of the present invention may also be used as a physiological or pharmacological reagent such as for detection of hANP and for determination of purification degree of hANP.

The method for immunological determination of hANP in accordance with the present invention is a method for quantitatively or semi-quantitatively determining hANP by immunological technique, using the hANP antibody of the present invention. As such a method for immunological determination which is applicable to the present invention, any method heretofore known to be as immunological technique using an antigen-antibody reaction may be used and, such examples include enzyme immunoassay (EIA), radioimmunoassay (RIA) and passive hemagglutination (PHA). In each of the methods described above, there are known various techniques such as a competitive method, a sandwich method and a second antibody method, and any of these methods may be adopted. Details of these methods for immunological determination and their specific techniques are described in, for example, Ishikawa et al., "Enzyme Immunoassay", 1978, published by Igaku Shoin Co., Ltd.; Irie, "Radioimmunoassay", 1974, published by Kodansha Co., Ltd., etc.

The method for immunological determination is specifically explained below, by referring to radioimmunoassay.

A kit for the determination of hANP utilizing hANP antibody of the present invention is exemplified by:

(1) a kit for the determination of hANP, which comprises a hANP antibody, a second antibody (IgG antibody), a radioactive substance-labeled hANP, a standard hANP, a precipitation accelerator (for B/F separation) and a buffer solution; and

(2) a kit for the determination of hANP, which comprises an insoluble carrier to which hANP antibody has been immobilized, a radioactive substance-labeled hANP, a standard hANP and a buffer solution.

In the kits stated above, the standard hANP is used to prepare a calibration curve described hereinafter and in general, it may be a lyophilized $\alpha$-hANP. The standard hANP may be used in the form of solution in a buffer. In this case, the solution is serially diluted at 4 to 5 stages of concentrations, for example, in the range of 1 to 10,000 pg/0.1 ml, which are used for preparation of the calibration curve.

A preferred example of the radioactive substance-labeled hANP is $^{125}$I-labeled $\alpha$-hANP. Where $^{125}$I is employed as a radioactive substance, a specific radioactivity of less than about 100 TBq per mmol of $\alpha$-hANP is appropriate. The radioactive substance-labeled hANP can be prepared by labeling hANP in a conventional manner, for example, by chloramine T method, lactoperoxidase method using lactoperoxidase and hydrogen peroxide, etc.

The buffer solution as used in the kits described above are for use in the antigen-antibody reaction, so that it is particularly preferred to use a buffer solution having a pH value of 6 to 9 and a salt concentration of approximately 0.01 to 0.2 M. A specific example may be 0.1 M phosphate buffered saline having pH 7.

The second antibody is an antibody to IgG, preferably an antibody to IgG from animal of the same species from which the hANP antibody is derived. In addition, protein A or protein C obtained from Staphylococcus may also be used together with the second antibody.

Examples of the precipitation accelerator include dextran and polyethylene glycol. Of these, polyethylene glycol having a molecular weight of about 1,000 to about 10,000 is preferred. The precipitation accelerator may be previously mixed with the second antibody.

As the insoluble carrier to which the hANP antibody has been immobilized, there may be exemplified polysaccharides such as cellulose, dextran and Sephadex; plastics such as polystyrene and polypropylene; synthetic fibers, glass, ceramics, etc. and materials obtained by introducing functional groups such as amino group and carboxy group into the above-mentioned materials. A shape of these materials is not particularly limited but for convenience of operations for the determination, a microplate, a plastic tube, a plastic ball and the like are preferred. Immobilization of the hANP antibody to the insoluble carrier may be conducted in a conventional manner. Examples of the immobilization method include the adsorption technique, the ionic bond technique, the covalent bond technique (e.g., carbodiimide method, glutaraldehyde method, cyanogen bromide activation method, etc.) and the like.

The operation for determination in the case of using a second antibody is specifically explained below as one example of the method for immunological determination according to the present invention.

Firstly, a sample solution is preincubated with the hANP antibody. Then a radioactive substance-labeled hANP is subjected to delayed addition to the preincubated hANP antibody followed by further incubation. Thereafter, a suspension comprising a second antibody and a precipitation accelerator is added to the incubated mixture. The precipitates (antigen-antibody complex) and the supernatant are separated from each other and measured with their radioactivity, respectively. Subsequently, B/T value is determined from the radioactivity (B) of the precipitates and the total radioactivity (T) of the supernatant and the precipitates, and the content of hANP in the sample solution is determined on the basis of calibration curve previously prepared in a conventional manner. The calibration curve is obtained by determining B/T value through similar operations using a standard $\alpha$-hANP sample having known concentrations instead of the sample solution and plotting the B/T values depending on the concentrations of hANP.

The method for immunological determination of hANP according to the present invention is not limited to the radioimmunoassay described above but may also be performed by any of known immunological methods. For example, in the aforesaid radioimmunoassay, the antigen-antibody reaction is carried out in the liquid phase. However, the antigen-antibody reaction may be performed by using a hANP antibody-immobilized insoluble carrier and adding thereto a sample solution and a radioactive substance-labeled hANP. After B/F separation by rinsing, radioactivity of the radioactive substance-labeled hANP remained on the solid phase may be determined. Furthermore, the method of the present invention may be performed by the sandwich method using a radioactive substance-labeled hANP antibody in place of a radioactive substance-labeled hANP. Furthermore, the method of the present invention may also be performed by enzymeimmunoassay using an enzyme-labeled hANP and an enzyme labeled hANP antibody, in place of the radioactive substance-labeled hANP and the radioactive substance-labeled hANP antibody, respectively, and measuring its reaction degree (reaction yield, reaction velocity, etc.) between the enzyme and its substrate for determination of activity of the enzyme label. Examples of the combination of enzyme label and substrate include a combination of peroxidase, hydrogen peroxide and o-phenylenediamine, a combination of $\beta$-galactosidase and o-nitrophenylphosphate-$\beta$-D-galactoside, and a combination of alkali phosphatase and p-nitrophenylphosphate.

An example of the sample solution to be assayed by the method for immunological determination of hANP according to the present invention is blood sample collected from a human body for inspection, but the sample solution is not limited to blood sample.

The antibody of the present invention is characterized by reacting with hANP having Tyr (28) and a cyclic structure involving the disulfide bond between Cys (7) and Cys (23) and fragment from Cys (7) to Met (12) in the molecule. Therefore, in the case of determining hANP in a vital sample solution, the physiologically active hANP can be measured with high sensitivity and selectivity, without requiring any special operations for extraction.

The antibody of the present invention is particularly useful for determination of, for example, $\alpha$-hANP, $\alpha$-hANP (4-28) and $\alpha$-hANP (5-28). It is known that in the living body, $\alpha$-hANP shows its activity in the form of $\alpha$-hANP, $\alpha$-hANP (4-28) or $\alpha$-hANP (5-28). It is also possible to determine $\beta$-hANP by using the h-ANP antibody of the present invention. Therefore, the present invention is useful for investigating the physiological significance of hANP.

The present invention is explained in more detail, with reference to examples and experimental examples but is not deemed to be limited thereto.

Example 1

(1) Production of specific antisera:

(i) Preparation of immunogen

Hapten antigen was prepared by the water-soluble carbodiimide coupling procedure. After 3.5 mg of $\alpha$-hANP (1-28) and 29.4 mg of bovine thyroglobulin were dissolved in 2.0 ml of 10 mM PBS (pH 7.2), 1.0 ml of distilled water containing 35 mg of 1-cyclohexyl-3-(2-morpholinoethyl)carbodiimide metho-p-toluenesulfonate was added to the solution. The resulting mixture was gently stirred at room temperature for 20 hours. In the reaction solution, the buffer was exchanged using Centricon 10. After bacteria-free filtration, the $\alpha$-

hANP-bovine thyroglobulin conjugate was stored at -20°C.

(ii) Immunization

The α-hANP-bovine thyroglobulin conjugate obtained in (i) above was mixed with H37Ra complete adjuvant in equal volumes to prepare a W/O emulsion. Immunization was performed by subcutaneously injecting 100 μg of the conjugate per Japanese white rabbit at the palm. Supplemental booster was performed by intracutaneously injecting the same dose in the back at intervals of 2 weeks for several months. Several days after the final booster, blood was collected and anti-sera were obtained in a conventional manner.

The thus obtained α-hANP antibody has properties of: Ig class, IgG; molecular weight, 150,000 to 160,000; isoelectric point, 5.8-7.3.

(2) A radioactive substance-labeled α-hANP:

$^{125}$I-α-hANP (-74 Tbq/mmol) was prepared by the lactoperoxidase method.

(3) Cross reactivity test:

Cross reactivity was examined by preparing a dose-reaction curve of α-hANP (1-28) [x] and various fragments [y] using $^{125}$I-α-hANP (1-28) and calculating cross reactivity in terms of a ratio (%) of amount x to amount y for inhibiting 50% of the binding with $^{125}$I-α-hANP (1-28).

The assay was performed by delayed addition of $^{125}$I-α-hANP. Each of serial dilutions of standard α-hANP (1-28) and various fragments with buffer were prepared for RIA; 0.1 ml of each dilution and 0.1 ml of anti-α-hANP-sera also appropriately diluted were added to an assay tube, respectively, which was then settled at 4°C for 20 hours. Then, 0.1 ml of $^{125}$I-α-hANP (1-28) having radioactivity of about 10,000 cpm was added thereto and the mixture was then settled at 4°C for further 20 hours. B/F separation was performed by adding 1.0 ml of a precipitation reagent containing goat-derived anti-rabbit IgG sera to the settled mixture and then settling at 4°C for 15 minutes. The radioactivity of the precipitates was measured with a gamma counter.

The results are shown in Table 1.

## Table 1

| Peptide | Cross Reactivity (%) |
|---|---|
| α-hANP (1-28) | 100 |
| " (1-10) | <0.001 |
| " (7-28) | 112 |
| " (5-28) | 110 |
| " (5-27) | <0.002 |
| " (13-28) | 0.01 |
| " (18-28) | <0.001 |

| | |
|---|---|
| Acm-α-hANP(a) | 33 |
| $Met(O)^{12}$-α-hANP | 142 |
| β-hANP | 108 |
| α-rANP (1-28)(b) | 64 |
| " (5-27)(c) | <0.002 |
| " (5-25)(d) | <0.002 |
| γ-rANP(e) | 13 |
| BNP(f) | 0.02 |
| Endothelin | <0.002 |
| ACTH(g) | <0.002 |
| Angiotensin II | <0.002 |
| Vasopressin | <0.002 |
| Osteocalcin | <0.002 |

(a): Acm-α-hANP indicates s-acetoamide methyl α-hANP.

(b), (c) and (d): α-rANPs (1-28), (5-27) and (5-25) indicate fragments of amino acids 1-28, 5-27 and 5-25 in the amino acid sequence of α type rat ANP, respectively [Biochem. Biophys. Res. Comnun., 121, 585 (1984); Science, 223, 67 (1984)].

(e): γ-rANP indicates γ type rat ANP [Nature, 312, 152 (1984)].

(f): BNP indicates a brain natriuretic peptide [Nature, 332, 78-81 (1988)].

(g): ACTH indicates an adrenocorticotropic hormone [Nature, 235, 114 (1972)].

8

As understood from Table 1, the $\alpha$-hANP antibody reacts with $\alpha$-hANPs (1-28), (7-28) and (5-28), Met-(0)$^{12}$-$\alpha$-hANP and $\beta$-hANP, whereas it does not react with $\alpha$-hANPs (1-10), (5-27), (13-28) and (18-28). From results shown in Table 1, it is revealed that the $\alpha$-hANP antibody is capable of recongnizing Tyr (28) and the cyclic structure involving the disulfide bond between Cys (7) and Cys (23) and fragment from Cys (7) to Met (12).

Example 2

(1) Preparation of reagents:

(i) $\alpha$-hANP antibody

Rabbit-derived hANP antisera prepared in a manner similar to Example 1 were used.

(ii) Standard $\alpha$-hANP

Purified $\alpha$-hANP solution (2560 pg/ml) was prepared.
Upon use, the solution was serially diluted with buffer to adjust to 2560, 1280, 640, 320, 160, 80, 40, 20 and 10 pg/ml.

(iii) A radioactive substance-labeled $\alpha$-hANP

$^{125}$I-$\alpha$-hANP (-74 TBq/mmol) prepared in a manner similar to Example 1 was used.

(iv) Second antibody preparations

Its composition was as follows.
Precipitation accelerator: PEG6000
Second antibody: goat-derived anti-rabbit-IgG antibody

(v) Buffer

0.15 M sodium chloride-supplemented 10 mM phosphate buffer (pH 7.0) was used.

(2) Method of measurement:

After 0.1 ml of standard $\alpha$-hANP solution or sample solution and 0.1 ml of hANP antisera were stirred, the mixture was settled at 2 to 8°C for 20 hours. Then, 0.1 ml of $^{125}$I-$\alpha$-hANP was added to the mixture. After stirring, the mixture was settled at 2 to 8°C for 20 hours. To the reaction solution was added 1.0 ml of second antibody. After stirring, the mixture was settled at 2 to 8°C for 15 minutes. Thereafter, centrifugation was performed at 2 to 8°C for 15 minutes under conditions of 3,000 rpm to achieve B/F separation. The radioactivity of the precipitates and the supernatant was measured and B/T value was calculated from the measurement data. A calibration curve was prepared from the measurement data of standard $\alpha$-hANP solution. Based on the calibration curve, the content of hANP in the sample solution was determined from the measurement data of the sample solution.

(3) Results:

(i) Sensitivity

Sensitivity was examined in the 2S.D. lower limit of binding % in standard solution (buffer) having zero concentration. As the result, the sensitivity was less than 10 pg/ml.

(ii) Plasma concentration in healthy donor

The concentration of hANP in plasma collected from healthy donor was examined. The results are shown in Table 2.

## Table 2

| Sample No. | Measurement Data (pg/ml) |
|---|---|
| 1 | 110.4 |
| 2 | 101.5 |
| 3 | 89.2 |
| 4 | 67.8 |
| 5 | 67.1 |
| 6 | 51.4 |
| 7 | 48.4 |
| 8 | 45.9 |
| 9 | 45.4 |
| 10 | 43.2 |
| 11 | 40.2 |
| 12 | 38.6 |
| 13 | 37.8 |
| 14 | 37.5 |
| 15 | 36.6 |
| 16 | 35.2 |

– cont'd –

EP 0 401 006 B1

| | |
|---|---|
| 17 | 35.0 |
| 18 | 32.6 |
| 19 | 31.2 |
| 20 | 30.7 |
| 21 | 29.8 |
| 22 | 29.5 |
| 23 | 29.5 |
| 24 | 28.8 |
| 25 | 25.8 |
| 26 | 25.5 |
| 27 | 25.2 |

The mean ANP concentration in plasma from healthy donor according to the method of the present invention was 45.2 ±4.5 pg/ml (mean ± SEM). This value is close to the values 37.7 ±7.0 pg/ml and 23.0 ±2.5 pg/ml obtained according to a $C_{18}$ column method [Biochem. Biophys. Res. Commun., 129, 439-446 (1985)] and an affinity column extraction method [J. Clin. Invest., 76, 1705-1709 (1985), respectively.

**Claims**

1. A human atrial natriuretic polypeptide (hANP) polyclonal and/or monoclonal antibody, which is capable of recognizing at least Tyr at the 28-position, the disulfide bond between Cys at the 7-position and Cys at the 23-position and the fragment from Cys at the 7-position to Met at the 12-position in the hANP molecule.

2. An hANP antibody according to Claim 1, which reacts with $\alpha$-hANP and its fragments of amino acids 5-28 and 7-28 in an amino acid sequence of $\alpha$-hANP.

3. An hANP antibody according to Claim 1, which does not react with fragments of amino acids 1-10, 13-28, 18-28 and 5-27 in an amino acid sequence of $\alpha$-hANP.

4. An hANP antibody according to Claim 1, which is an antiserum from an animal immunized with $\alpha$-hANP or its fragments.

5. An hANP antibody according to Claim 4, which has the following properties: Ig class, IgG; molecular weight, 150,000 to 160,000; and isoelectric point, 5.8-7.3.

6. A method for the immunological determination of hANP, which uses an hANP antibody according to any preceding Claim.

7. A method according to Claim 6, which is carried out according to enzyme immunoassay, radioimmuno assay or passive hemagglutination.

8. A kit for the immunological determination of hANP, which comprises an hANP antibody according to any preceding Claim.

11

9. A kit according to Claim 8, which further comprises a second antibody, a radioactive substance-labelled hANP, a standard hANP, a precipitation accelerator and a buffer solution.

10. A kit according to Claim 8, which further comprises an insoluble carrier having said hANP antibody immobilized thereon, a radioactive substance-labelled hANP, a standard hANP and a buffer solution.

11. A method of making a preparation comprising a human atrial natriuretic polypeptide (hANP) antibody, which antibody is capable of recognising the following sites, namely (1) at least Tyr at the 28-position, (2) the disulphide bond between the Cys at the 7-position and Cys at the 23-position and (3) the fragment from Cys at the 7-position to Met at the 12-position in the hANP molecule, which method comprises the steps of
(i) immunizing an animal with hANP as antigen;
(ii) producing a medium containing polyclonal or monoclonal antibody; and
(iii) testing the medium for the presence of an antibody which recognises the said sites (1), (2) and (3).

12. An antiserum containing a human atrial natriuretic polypeptide (hANP) antibody, which is capable of recognizing at least Tyr at the 28-position, the disulfide bond between Cys at the 7-position and Cys at the 23-position and the fragment from Cys at the 7-position to Met at the 12-position in the hANP molecule.

13. Use of an antibody according to claim 1 or antiserum according to claim 12 for detection of hANP.

**Patentansprüche**

1. Polyklonaler und/oder monoklonaler Antikörper gegen ein menschliches atriales natriuretisches Polypeptid (hANP), der zumindest Tyr an der 28-Position, die Disulfid-Bindung zwischen Cys an der 7-Position und Cys an der 23-Position und das Fragment von Cys an der 7-Position bis zu Met an der 12-Position im hANP-Molekül erkennen kann.

2. Antikörper gegen hANP nach Anspruch 1, der mit α-hANP und seinen Fragmenten der Aminosäuren 5-28 und 7-28 in einer Aminosäuresequenz von α-hANP reagiert.

3. Antikörper gegen hANP nach Anspruch 1, der mit Fragmenten der Aminosäuren 1-10, 13-28, 18-28 und 5-27 in einer Aminosäuresequenz von α-hANP nicht reagiert.

4. Antikörper gegen hANP nach Anspruch 1, der ein Antiserum aus einem mit α-hANP oder seinen Fragmenten immunisierten Tier ist.

5. Antikörper gegen hANP nach Anspruch 4, der die folgenden Eigenschaften aufweist: Ig-Klasse: IgG; Molekulargewicht: 150.000 bis 160.000; isoelektrischer Punkt: 5,8-7,3.

6. Verfahren zur immunologischen Bestimmung von hANP, das die Verwendung eines Antikörpers gegen hANP nach einem der vorhergehenden Ansprüche vorsieht.

7. Verfahren nach Anspruch 6, das gemäß einem Enzym-Immunoassay, Radioimmuno-Assay oder passiver Hämagglutination erfolgt.

8. Satz zur immunologischen Bestimmung von hANP, der einen Antikörper gegen hANP nach einem der vorhergehenden Ansprüche umfaßt.

9. Satz nach Anspruch 8, der weiters einen zweiten Antikörper, ein mit einer radioaktiven Substanz markiertes hANP, ein Standard-hANP, einen Fällungsbeschleuniger und eine Pufferlösung umfaßt.

10. Satz nach Anspruch 8, der weiters einen unlöslichen Träger mit dem darauf immobilisierten Antikörper gegen hANP, ein mit einer radioaktiven Substanz markiertes hANP, ein Standard-hANP und eine Pufferlösung umfaßt.

**11.** Verfahren zur Herstellung eines Präparats, das einen Antikörper gegen menschliches atriales natriuretisches Polypeptid (hANP) umfaßt, der die folgenden Stellen erkennen kann: (1) zumindest Tyr an der 28-Position, (2) die Disulfid-Bindung zwischen dem Cys an der 7-Position und Cys an der 23-Position und (3) das Fragment von Cys an der 7-Position bis zu Met an der 12-Position im hANP-Molekül, welches Verfahren die folgenden Schritte umfaßt:

(i) Immunisieren eines Tiers mit hANP als Antigen;

(ii) Erzeugen eines Mediums, das polyklonalen oder monoklonalen Antikörper enthält; und

(iii) Prüfen des Mediums hinsichtlich der Gegenwart eines Antikörpers, der die Stellen (1), (2) und (3) erkennt.

**12.** Antiserum, das einen Antikörper gegen menschliches atriales natriuretisches Polypeptid (hANP) enthält, der zumindest Tyr an der 28-Position, die Disulfid-Bindung zwischen Cys an der 7-Position und Cys an der 23-Position und das Fragment von Cys an der 7-Position bis zu Met an der 12-Position im hANP-Molekül erkennen kann.

**13.** Verwendung eines Antikörpers nach Anspruch 1 oder Antiserums nach Anspruch 12 zum Nachweis von hANP.

**Revendications**

**1.** Anticorps monoclonal et/ou polyclonal pour le polypeptide natriurétique atrial humain (hANP), qui est capable de reconnaître au moins Tyr à la position 28, la liaison disulfure entre Cys à la position 7 et Cys à la position 23 et le fragment de Cys à la position 7 à Met à la position 12 dans la molécule de hANP.

**2.** Anticorps contre hANP selon la revendication 1, qui réagit avec α-hANP et ses fragments d'acides aminés 5 à 28 et 7 à 28 dans une séquence d'acides aminés de α-hANP.

**3.** Anticorps contre hANP selon la revendication 1, qui ne réagit pas avec les fragments d'acides aminés 1 à 10, 13 à 28, 18 à 28 et 5 à 27 dans une séquence d'acides aminés de α-hANP.

**4.** Anticorps contre hANP selon la revendication 1, qui est un antisérum d'un animal immunisé avec α-hANP ou ses fragments.

**5.** Anticorps contre hANP selon la revendication 4, qui a les propriétés suivantes : classe Ig, IgG; poids moléculaire, 150 000 à 160 000; et point isoélectrique, 5,8-7,3.

**6.** Méthode de détermination immunologique de hANP, qui utilise un anticorps contre hANP selon l'une quelconque des revendications précédentes.

**7.** Méthode selon la revendication 6, qui est mise en oeuvre selon un immunodosage, un radioimmunodosage ou une hémagglutination passive d'enzyme.

**8.** Kit de détermination immunologique de hANP, qui comprend un anticorps contre hANP selon l'une quelconque des revendications précédentes.

**9.** Kit selon la revendication 8, qui comprend de plus un second anticorps, un hANP marqué par une substance radioactive, un hANP standard, un accélérateur de précipitation et une solution tampon.

**10.** Kit selon la revendication 8, qui comprend de plus un support insoluble ayant ledit anticorps contre hANP immobilisé sur celui-ci, un hANP marqué par une substance radioactive, un hANP standard et une solution tampon.

**11.** Méthode de fabrication d'une préparation comprenant un anticorps contre le polypeptide natriurétique atrial humain (hANP), lequel anticorps est capable de reconnaître les sites suivants, à savoir (1) au moins Tyr à la position 28, (2) la liaison disulfure entre Cys à la position 7 et Cys à la position 23 et (3) le fragment de Cys à la position 7 à Met à la position 12 dans la molécule de hANP, laquelle méthode comprend les étapes de

13

EP 0 401 006 B1

(i) immuniser un animal avec hANP comme antigène;
(ii) produire un milieu contenant un anticorps polyclonal ou monoclonal; et
(iii) tester le milieu pour la présence d'un anticorps qui reconnaît lesdits sites (1), (2) et (3).

12. Antisérum contenant un anticorps contre le polypeptide natriurétique atrial humain (hANP), qui est capable de reconnaître au moins Tyr à la position 28, la liaison disulfure entre Cys à la position 7 et Cys à la position 23 et le fragment de Cys à la position 7 à Met à la position 12 dans la molécule hANP.

13. Utilisation d'un anticorps selon la revendication 1 ou d'un antisérum selon la revendication 12 pour la détection de hANP.